Europäisches Patentamt

European Patent Office

Office européen des brevets

(11) Veröffentlichungsnummer: **0 090 966**
**B1**

# EUROPÄISCHE PATENTSCHRIFT

(45) Veröffentlichungstag der Patentschrift:
18.12.85

(51) Int. Cl.⁴: **G 01 N 33/557**

(21) Anmeldenummer: **83102566.3**

(22) Anmeldetag: **15.03.83**

(54) Verfahren zur Antigen-Bestimmung.

(30) Priorität: **05.04.82 DE 3212658**

(43) Veröffentlichungstag der Anmeldung:
**12.10.83 Patentblatt 83/41**

(45) Bekanntmachung des Hinweises auf die Patenterteilung:
**18.12.85 Patentblatt 85/51**

(84) Benannte Vertragsstaaten:
**AT BE CH FR GB IT LI NL**

(56) Entgegenhaltungen:
**GB - A - 1 540 098**
**GB - A - 1 590 525**
**GB - A - 1 599 543**

(73) Patentinhaber: **AMERICAN HOSPITAL SUPPLY CORPORATION, One American Plaza, Evanston, IL 60201 (US)**

(72) Erfinder: **Danninger, Josef, Finkenstrasse 27a, D-8032 Gräfelfing (DE)**
Erfinder: **Spaethe, Reiner, Dr. med., Prinz-Karl-Strasse 41, D-8130 Starnberg-Söcking (DE)**

(74) Vertreter: **Brose, D. Karl, Dipl.-Ing., Wiener Strasse 2, D-8023 München-Pullach (DE)**

## Beschreibung

Die Erfindung bezieht sich auf ein Verfahren zur Bestimmung des Antigen-Gehalts einer Probe durch die Antigen-Antikörper-Reaktion. Sie hat auch ein Verdünnungsmittel für die Antigen- bzw. Antikörper-Reaktionskomponente zur Durchführung dieses Verfahrens zum Gegenstand.

Die Bestimmung von diagnostisch relevanten Parametern mittels Antigenen in Proben, wie Serum, Plasma oder Liqour, durch die immunologische oder Antigen-Antikörper-Reaktion, die fotometrisch beispielsweise durch Nephelometrie oder Turbidimetrie ausgewertet wird, bedingt oftmals eine spezielle Vorbereitung der beiden Reaktionskomponenten. Üblicherweise wird dabei die eine Reaktionskomponente, die die Probe enthält, deren Antigen-Gehalt bestimmt werden soll, und die andere Komponente, die den Antikörper enthält, also in der Regel durch das entsprechende Antiserum gebildet wird, mit physiologischer Kochsalzlösung, die zusätzlich 4% Polyäthylenglykol (mittleres Molekulargewicht 6000) enthält, verdünnt.

Auch ist es beim Radioimmunoassay bekannt, die Antigen-Antikörper-Reaktion in einer polyäthylenglykolhaltigen Kochsalzlösung durchzuführen, die mit Phosphat gepuffert ist (GB-A-1540098).

Der Gehalt an Polyäthylenglykol dient hauptsächlich dazu, die bei der Reaktion entstehenden Antigen-Antikörper-Komplexe an einer Ausflokkung, d.h. an der Bildung eines Niederschlags in der durch die Vereinigung der beiden Reaktionskomponenten gebildeten Testlösung während der Antigen-Bestimmung, also des Messvorgangs zu verhindern. Diese Eigenschaft des Polyäthylenglykols ist seit längerer Zeit bekannt und stellt für diese Messungen eine Grundanforderung dar, da sonst nicht alle gebildeten Antigen-Antikörper-Komplexe während der Messung erfasst werden können.

Bei der Bestimmung von Immunglobulinen durch Laserturbidimetrie nach «Arbeitsanleitung für das Lasermed-Turbidometer (pm 4) zur Bestimmung der Immunglobuline IgA, IgG, IgM mit ATAB-Antiseren, Firma AHS Deutschland GmbH, Bereich Merz und Dade, München», 1982, wurde festgestellt, dass verschiedene physiologische Kochsalzlösungen mit 4 Gew.-% Polyäthylenglykol, obwohl sie jeweils nach derselben Rezeptur, mit denselben Chemikalien und von der gleichen Person hergestellt worden waren, unterschiedliche Messsignale am Laserturbidometer während der Analyse ergaben. Es konnten also keine hinreichend reproduzierbaren Ergebnisse erhalten werden. Besonders gestört wurde dabei die sogenannten Kinetik-Methode nach der erwähnten Arbeitsanleitung, bei der nach einer Vorreaktion von beispielsweise 10 s die Geschwindigkeit der Antigen-Antikörper-Reaktion gemessen und dann der Antigen-Gehalt bestimmt wird. Im Gegensatz zur sogenannten «Fix point-Methode» nach der erwähnten Arbeitsanleitung, d.h. der Endpunktmessung nach einer Reaktionsdauer von beispielsweise 30 min, braucht man bei der erwähnten Kinetik-Methode also nicht den gesamten Verlauf der Antigen-Antikörper-Reaktion bis zu Ende abzuwarten.

Die Erfindung löst die Aufgabe, ein Verfahren bzw. ein Verdünnungsmittel bereitzustellen, mit dem der Antigen-Gehalt einer Probe selbst mit der Kinetik-Methode reproduzierbar und exakt bestimmbar ist.

Das Antikörpermolekül besitzt eine polyvalente ionogene und deshalb stark strukturierte Form, während Polyäthylenglykol nicht-ionogen und deshalb unstrukturiert ist. Beim Zusammengeben der beiden Reaktionskomponenten kommt es deshalb erst nach geraumer Zeit zu einer gewissen homogenen Verteilung, welche einigermassen reproduzierbare Analysenergebnisse liefert. Das erfindungsgemässe Verfahren beruht nun darauf, eine rasche und homogene Verteilung der beiden Reaktionskomponenten dadurch zu erzielen, dass die polyäthylenglykolhaltige, physiologische Kochsalzlösung zusätzlich mit bestimmten Puffersubstanzen versetzt wird, die die strukturierte Form des Antikörpermoleküls durch Ladungsausgleich weitgehend aufhebt. Als Puffersubstanzen werden erfindungsgemäss Glycin-, Glycylglycin-, Acetat-, Zitrat-, N,N-Bis-(2-hydroxyäthyl)glycin-, Histidin/HCl-, 2-Amino-2-(hydroxymethyl)-1,3-propandiol- oder 5,5-Dialkylbarbitursäure-Puffer verwendet. Oberraschenderweise hat sich herausgestellt, dass die Geschwindigkeit der Antigen-Antikörper-Reaktion insbesondere von der chemischen Zusammensetzung der Puffersubstanz abhängig ist, und zwar weit mehr als von der Konzentration der Puffersubstanz oder dem pH-Wert. Von besonderer Bedeutung bei der Erfindung ist weiter, dass nicht solche Puffersubstanzen gewählt werden, die eine schnellst mögliche Reaktionsgeschwindigkeit zulassen, sondern solche Puffersubstanzen, die zu einer sich über einen längeren Zeitraum erstreckenden konstanten Anfangsreaktionsgeschwindigkeit führen. Durch diese Eigenschaft der erfindungsgemäss einsetzbaren Puffersubstanzen wird nämlich die Reaktion gewissermassen gestreckt, so dass sich ein linearer Verlauf über einen relativ langen Zeitraum ergibt. Dadurch wird die Genauigkeit der Ergebnisse der Kinetik-Methode beträchtlich verbessert. Werden beispielsweise 20 mmol/15,5-Diäthylbarbitursäure, pH 7,5, als Puffer zu der physiologischen, 4 Gew.-% polyäthylenglykol-haltigen Kochsalzlösung zugesetzt, so wird nach der Vereinigung der beiden Reaktionskomponenten eine Vorreaktionszeit von beispielsweise 10 s abgewartet, an die sich eine Messzeit von beispielsweise 30 s anschliesst. Der Zeitraum von 30 s stellt dann eine auch für sehr präzise Messergebnisse ausreichende Messgrundlage für die Antigen-Bestimmung dar. Weitere Ausführungsformen des erfindungsgemässen Verfahrens sind in den Ansprüchen 2 bis 6 beschrieben.

## Patentansprüche

1. Verfahren zur Bestimmung des Antigen-Gehalts einer Probe durch die Antigen-Antikörper-Reaktion, wobei zumindest eine der beiden Reaktionskomponenten mit einer physiologischen Kochsalzlösung, die Polyäthylenglykol und eine Puffersubstanz enthält, verdünnt wird, dann die beiden Reaktionskomponenten vereinigt werden und die Antigen-Antikörper-Reaktion fotometrisch bestimmt wird, dadurch gekennzeichnet, dass die Puffersubstanz ein Glycin-, Glycylglycin-, Acetat-, Zitrat-, N,N-Bis-(2-hydroxyäthyl)-glycin-, Histidin/HCl-, 2-Amino-2-(hydroxymethyl)-1,3-propandiol- oder 5,5-Dialkylbarbitursäure-Puffer ist.

2. Verfahren nach Anspruch 1, dadurch gekennzeichnet, dass die Barbitursäure 5,5-Diäthylbarbitursäure ist.

3. Verfahren nach Anspruch 1 oder 2, dadurch gekennzeichnet, dass die Konzentration der Puffersubstanz in der Kochsalzlösung 5 bis 200 mmol/l, vorzugsweise 20 bis 50 mmol/l beträgt.

4. Verfahren nach einem der vorstehenden Ansprüche, dadurch gekennzeichnet, dass die Konzentration des Polyäthylenglykols in der Kochsalzlösung 2 bis 6 Gew.-%, vorzugsweise etwa 4 Gew.-% und das mittlere Molekulargewicht des Polyäthylenglykols 4000 bis 12000, vorzugsweise etwa 6000 beträgt.

5. Verfahren nach einem der vorstehenden Ansprüche, dadurch gekennzeichnet, dass der pH-Wert der die Puffersubstanz enthaltenden, polyäthylenglykolhaltigen physiologischen Kochsalzlösung 4,0 bis 9,0 und vorzugsweise 6,5 bis 8 beträgt.

6. Verfahren nach einem der vorstehenden Ansprüche, dadurch gekennzeichnet, dass eine fotometrische Bestimmung des kinetischen Verlaufs der Antigen-Antikörper-Reaktion durchgeführt wird.

7. Polyäthylenglykol und eine Puffersubstanz enthaltende physiologische Kochsalzlösung als Verdünnungsmittel wenigstens einer der beiden Reaktionskomponenten der Antigen-Antikörper-Reaktion zur fotometrischen Bestimmung des Antigen-Gehalts, dadurch gekennzeichnet, dass die Puffersubstanz ein Glycin-, Glycylglycin-, Acetat-, Zitrat-, N,N-Bis-(2-hydroxyäthyl)-glycin-, Histidin/HCl-, 2-Amino-2-(hydroxymethyl)-1,3-propandiol- oder 5,5-Dialkylbarbitursäure-Puffer ist.

## Claims

1. A method for determination of the antigen content of a sample by the antigen-antibody reaction, comprising deluting at least one of the two reaction components with a physiological saline solution, which contains polyethyleneglycol and a buffer substance, thereafter combining both reaction components and determining the antigen-antibody-reaction photometrically, characterized in that the buffer substance is a glycine, glycylglycine, acetate, citrate, N,N-bis-(2-hydroxyethyl)-glycine, histidine/HCl, 2-amino-2-(hydroxymethyl)-1,3-propanol or 5,5-dialkyl-barbituric acid buffer.

2. A method according to claim 1, characterized in that the barbituric acid is 5,5-diethyl-barbituric acid.

3. A method according to claim 1 or 2, characterized in that the concentration of the buffer substance in the saline solution is 5 to 200 mmol/l, preferably 20 to 50 mmol/l.

4. A method according to one of the preceding claims, characterized in that the concentration of the polyethyleneglycol in the saline solution is 2 to 6% by weight, preferably about 4% by weight, and the average molecular weight of the polyethyleneglycol is 4000 to 12.000, preferably about 6000.

5. A method according to one of the preceding claims, characterized in that the pH-value of the physiological saline solution, containing the buffer substance and the polyethyleneglycol, is 4,0 to 9,0, preferably 6,5 to 8.

6. A method according to one of the preceding claims, characterized in that a photometrical determination of the kinetic progression of the antigen-antibody-reaction is performed.

7. A polyethylene and a buffer substance containing physiological saline solution as diluent of at least one of the two reaction components of the antigen-antibody-reaction for the photometrical determination of the antigen content, characterized in that the buffer substance is a glycine, glycylglycine, acetate, citrate, N,N-bis-(2-hydroxyethyl)-glycine, histidine/HCl, 2-amino-2-(hydroxymethyl)-1,3-propanol or 5,5-dialkyl-barbituric acid buffer.

## Revendications

1. Procédé de dosage de la teneur en antigène d'un échantillon par la réaction antigène-anticorps, en diluant l'un au moins des deux composants réactionnels avec une solution physiologique de chlorure de sodium qui renferme du polyéthylèneglycol et une substance tampon, puis en réunissant les deux composants réactionnels et en dosant photométriquement la réaction antigène-anticorps, caractérisé en ce que la substance tampon est un tampon à base de glycine, de glycylglycine, d'acétate, de citrate, de N,N-bis-(2-hydroxyéthyl)-glycine, d'histidine/HCl, de 1-amino-2-(hydroxyméthyl)-1,3-propandiol ou d'acide 5,5-dialcoylbarbiturique.

2. Procédé selon la revendication 1, caractérisé en ce que l'acide barbiturique est l'acide 5,5-diéthylbarbiturique.

3. Procédé selon l'une des revendications 1 et 2, caractérisé en ce que la concentration en substance tampon de la solution de chlorure de sodium est de 5 à 200 mmol/l, de préférence de 20 à 50 mmol/l.

4. Procédé selon l'une quelconque des revendications précédentes, caractérisé en ce que la concentration en polyéthylèneglycol de la solution de chlorure de sodium est de 2 à 6% en poids, de préférence d'environ 4% en poids, et en ce que le poids moléculaire moyen de polyéthylèneglycol est de 4000 à 12000, de préférence environ 6000.

5. Procédé selon l'une quelconque des revendi- cations précédentes, caractérisé en ce que la valeur du pH de la solution physiologique de chlorure de sodium renfermant du polyéthylèneglycol, contenant la substance-tampon, est comprise entre 4,0 et 9,0, de préférence entre 6,5 et 8.

6. Procédé selon l'une quelconque des revendications précédentes, caractérisé en ce qu'on effectue un dosage photométrique du déroulement cinétique de la réaction antigène-anticorps.

7. Solution physiologique de chlorure de sodium renfermant du polyéthylèneglycol et une substance tampon utilisée comme diluant de l'un au moins des deux composants réactionnels de la réaction antigène-anticorps aux fins de dosage photométrique de la teneur en antigène, caractérisée en ce que la substance tampon est un tampon à base de glycine, de glycylglycine, d'acétate, de citrate, de N,N-bis (2-hydroxyéthyl)-glycine, d'histidine/HCl, de 2-amino-2-(hydroxyéthyl)-1,3-propandiol ou d'acide 5,5-dialcoylbarbiturique.